## Europäisches Patentamt
## European Patent Office
## Office européen des brevets

(11) Veröffentlichungsnummer: **0 436 121 A1**

# EUROPÄISCHE PATENTANMELDUNG

(21) Anmeldenummer: 90122713.2

(51) Int. Cl.⁵: **A61B 9/00, A61B 5/22**

(22) Anmeldetag: 28.11.90

(30) Priorität: 01.12.89 DE 3939790

(43) Veröffentlichungstag der Anmeldung:
**10.07.91 Patentblatt 91/28**

(84) Benannte Vertragsstaaten:
**DE DK FR SE**

(71) Anmelder: **Drägerwerk Aktiengesellschaft**
**Moislinger Allee 53-55**
**W-2400 Lübeck 1(DE)**

(72) Erfinder: **Wallroth, Carl F., Dr.**
**Stresemannstrasse 1**
**W-2400 Lübeck(DE)**
Erfinder: **Jaklitsch, Roman R., Dr.**
**Wakenitzmauer 21**
**W-2400 Lübeck(DE)**

(54) **Vorrichtung zur Messung des Relaxationsgrades.**

(57) Zur Erfassung von durch Elektrostimulation induzierten Muskelkontraktionen wird mindestens ein Messwertaufnehmer (13,14) verwendet, welcher an einem stimulierten Fingerglied befestigbar ist und dessen Ausgangssignal ein Mass für den zu bestimmenden Relaxationsgrad darstellt.

Zur Erlangung verstärkter Ausgangssignale und zur Ausfilterung von Artefakten ist dem Daumen und dem kleinen Finger der Hand je ein Messwertaufnehmer (13,14) zugeordnet, so dass der Relaxationsgrad aus den kombinierten Ausgangssignalen beider Messwertaufnehmer (13,14) bestimmt werden kann.

EP 0 436 121 A1

# VORRICHTUNG ZUR MESSUNG DES RELAXATIONSGRADES

Die Erfindung betrifft eine Vorrichtung zur Messung des Relaxationsgrades durch Erfassung der durch Elektrostimulation induzierten Muskelkontraktionen im Fingerbereich unter Verwendung von mindestens einem Meßwertaufnehmer, welcher an dem stimulierten Fingerglied befestigbar ist und dessen Ausgangssignal ein Maß für den zu bestimmenden Relaxationsgrad darstellt.

In der Veröffentlichung von W. Friesdorf, M. Schultz und H.-H. Mehrkens "Eine einfache Methode zur Bestimmung und Registrierung des Relaxationsgrades" DEZ Anästhesie. Intensivtherapie. Notfallmedizin, Band 19, 1984, Seiten 78 - 80 ist angegeben, auf dem Handrücken eines narkotisierten Patienten einen Sensor anzubringen, der mit einem Finger, vorzugsweise mit dem Daumen,verbunden ist. Der Meßfühler besteht aus einem Stahlfederband, das einerseits auf dem Handrücken und andererseits am Daumen befestigt wird. Zwei auf beide Seiten des Stahlfederbandes geklebte Dehnungsmeßstreifen erfassen die Verformung. Dabei wird in einer Brückenschaltung ein elektrisches Signal erzeugt, welches angezeigt oder aufgezeichnet werden kann. Stimuliert man durch elektrische Impulse einen der Daumenbewegung zugeordneten Nerv, so kommt es zu einer Kontraktion der vom Nerv versorgten Muskeln. Aus dem Eintreten bzw. der Stärke der Kontraktion, welche sich in der Auslenkung gegen die elastische Vorspannung des Sensorträgers ausdrückt, kann auf den Relaxierungsgrad geschlossen werden. Bei vollständiger Relaxation tritt trotz elektrischer Reizung der die Muskelbewegung innervierenden Nerven keine Daumenbewegung ein. Die Überwachung der Relaxierung besitzt vor allem für die Narkoseausleitung große Bedeutung, da vor der Extubation zur Sicherstellung einer suffizienten Eigenatmung die Relaxierung genügend weit abgeklungen sein muß.

Ein Relaxometriesensor der eingangs genannten Art ist in der DE-PS 34 44 628 dargestellt. Der dort beschriebene Sensor weist einen Träger mit im wesentlichen rechteckigem Basisteil und damit einstückig verbundenem Zungenteil aus Kunststoff auf, wobei am Basisteil und am Zungenteil lösbare Verschlußelemente zur Fixierung am Handrücken und zur Befestigung an dem zu stimulierenden Finger angeordnet sind und bei dem der Meßwertaufnehmer aus Dehnungsmeßstreifen besteht, die auf der Ober- und Unterseite eines Einlageteils im Träger aufliegen.

Derartige vorbekannte Ausbildungen, bei denen der Relaxationsgrad aus der Bewegung eines einzelnen Fingers als Auslöseglied bestimmt wird, ergeben unter bestimmten Voraussetzungen keine hinreichend genauen Meßwerte, weil die Ausgangssignale, beispielsweise durch externe Berührungen, zusätzlich erzeugt werden und somit nicht von der Elektrostimulation und der damit verbundenen Muskelkontraktion herrühren.

Die Erfindung geht von der Aufgabenstellung aus, eine Vorrichtung der eingangs beschriebenen Art so zu verbessern, daß Artefakte, d.h. nicht über den Relaxationsgrad mit der Elektrostimulation verknüpfte Reizantworten bei der Bestimmung des Relaxationsgrades ausgeschaltet werden können. Ferner soll ein besonders einfacher und benutzerfreundlicher Sensoraufbau erzielt werden.

Zur Lösung dieser Aufgabenstellung ist vorgesehen, daß je ein dem Daumen und dem kleinen Finger der Hand zugeordneter Meßwertaufnehmer angeordnet ist, und daß der Relaxationsgrad aus den kombinierten Ausgangssignalen beider Meßwertaufnehmer bestimmt wird.

Im Gegensatz zu den vorbekannten Relaxometriesensoren, bei denen jeweils nur die Bewegung eines Fingers, vorzugsweise des Daumens, ausgewertet wird, erfolgt bei der Ausführungsform der Erfindung die Auswertung der Bewegungsabläufe zweier von verschiedenen Nervenästen (nervus ulnaris und medialis) stimulierten Fingerbewegungen, nämlich des Daumens und des kleinen Fingers. Durch diese Kombination lassen sich Reizantworten zur Bestimmung des Relaxationsgrades verstärken, indem die Ausgangssignale beider Meßwertaufnehmer additiv ausgewertet werden. Ferner ergibt sich die wichtige Möglichkeit, aus den beiden Signalen Artefakte herauszufiltern, wenn beispielsweise nur einer der beiden Finger ein Ausgangssignal erzeugt, welches u.U. von einer externen Berührung herrührt. Selbstverständlich müssen die Nerven beider Finger jeweils gleichzeitig bzw. in definiertem zeitlichem Abstand stimuliert werden.

Eine solche Vorrichtung zur Messung des Relaxationsgrades kann in der Weise aufgebaut werden, daß mechanisch getrennte, jedoch elektrisch gekoppelte Meßwertaufnehmer benutzt werden. Besonders zweckmäßig erscheint jedoch die Anwendung beider Meßwertaufnehmer im Rahmen eines eine Baueinheit bildenden Relaxometriesensors, wobei auf einem gemeinsamen Träger angeordnete, mechanischelektronische Meßwertaufnehmer Daumen und kleinen Finger umspannen.

Der gemeinsame Träger kann zweckmäßig aus einer Handgelenkmanschette und einem die Meßwertaufnehmer tragenden Handteil bestehen.

Außerdem erscheint es vorteilhaft, zwischen dem Handteil des Trägers und der Handgelenkmanschette ein flexibles Übergangsstück anzuordnen. Die Stimulationselektroden werden dabei zweckmäßig an der Handgelenkmanschette angeordnet.

Ein weiterer Vorteil kann gegebenenfalls dadurch erreicht werden, daß der Handteil aus miteinander verbundenen Handrücken- und Handinnenflächenteilen besteht, welche handschuhartige Durchtrittsaussparungen zur Aufnahme des Daumens und der Finger aufweisen. Bei einer solchen Ausbildung läßt sich der Relaxometriesensor in einfacher Weise über die Hand streifen und mit der Handgelenksmanschette fixieren.

Obwohl die Meßwertaufnehmer in ganz verschiedener Weise, beispielsweise auch als piezoelektrische Beschleunigungsaufnehmer, als Dehnungsmeßstreifen oder dergleichen ausgebildet sein können, erscheint es besonders zweckmäßig, die Meßwertaufnehmer als paarweise jeweils auf der Ober- und Unterseite eines elastisch verformbaren Trägers angeordnete Dehnungsmeßstreifen auszubilden.

Dabei kann es zweckmäßig sein, daß der mit dem Daumen und mit dem kleinen Finger zu verbindende Meßwertaufnehmer in seiner Halterung derart elastisch gestaltet ist, daß eine zur Messung des Relaxationsgrades ausreichende, der stimulierenden Auslenkung graduell entgegenwirkende mechanische Vorspannung auftritt. Eine solche Ausbildung wird bei auf der Ober- und Unterseite eines Trägermaterials liegenden Dehnungsmeßstreifen beispielsweise dadurch erreicht, daß der Träger aus Hart-Polyvenylchlorid in einer Dicke von 0,1 - 0,7 mm besteht.

Zur leichten Handhabung erscheint es vorteilhaft, am Handteil und/oder an der Handgelenkmanschette lösbare Schnellbefestigungselemente, insbesondere Klettverschlüsse, in der aus der DE-PS 34 44 628 bekannten Weise anzubringen, mit denen der Relaxometriesensor rasch angelegt und wieder abgenommen werden kann. In der Gesamtausbildung wird eine leicht zu handhabende Baueinheit angestrebt, die aseptischen Anforderungen genügt.

Die Auswertung der von den beiden Meßwertaufnehmern abgegebenen Ausganssignale erfolgt über an sich bekannte Elektronikschaltungen, wobei die Vorrichtung zur Elektrostimulation und die Auswerteelektronik schaltungsmäßig zusammengefaßt werden können.

Durch die Anwendung der Merkmale der Erfindung wird der Aufbau eines Relaxometriesensors ermöglicht, welcher verstärkte Ausgangssignale liefert, die von Störsignalen einwandfrei unterschieden werden können, so daß eine verläßliche Bestimmung des Relaxationsgrades möglich wird.

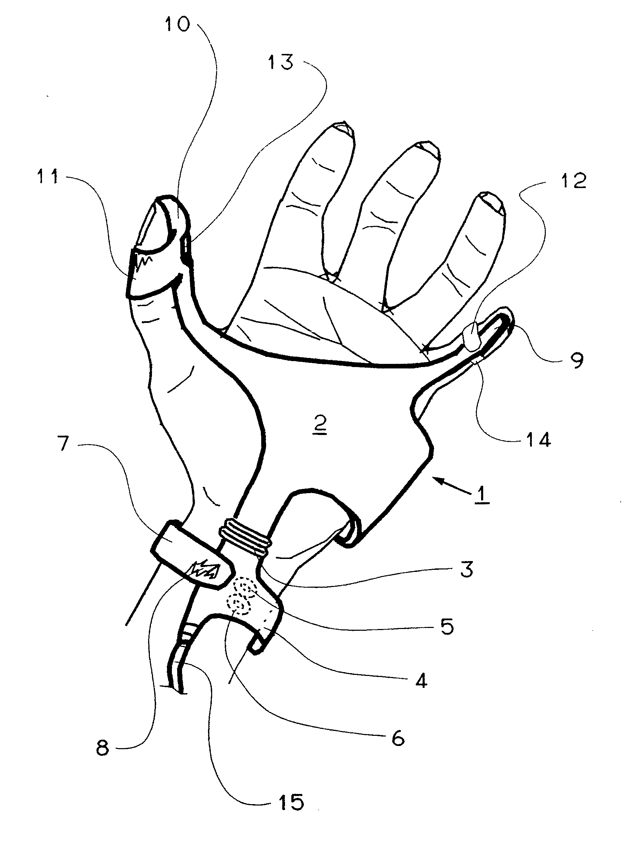

In der Zeichnung ist ein Ausführungsbeispiel des Gegenstandes der Erfindung schematisch dargestellt.

Man erkennt einen Träger 1, bei dem ein Handteil 2 über ein flexibles Übergangsstück 3 mit einer Handgelenkmanschette 4 bewegbar verbunden ist. An der Handgelenkmanschette sind in entsprechender Lageorientierung Stimulationselektroden 5,6 zur Stimulierung der Nervenäste angebracht. Die Handgelenkmanschette 4 ist über einem Zungenteil 7 an einem Klettverschluß 8 befestigbar.

In gleicher Weise sind die elastischen Auslenkteile 9 und 10 des Handteils 2 mit schlaufenförmigen Klettverschlüssen 11,12 am Daumen und am kleinen Finger befestigbar. Auf der Oberseite und Unterseite der Auslenkteile 9,10 befinden sich jeweils paarweise angeordnete Dehnungsmeßstreifen, welche die beiden Meßwertaufnehmer 13,14 bilden. Die Ausgangssignale der Meßwertaufnehmer 13,14 werden über in der Zeichnung nicht dargestellte Verbindungsleitungen in ein Anschlußkabel 15 des Relaxometriesensors eingeführt, welches auch die Zuleitungen zu den Stimulationselektroden 5,6 integriert.

Die Ausgangssignale der Meßwertaufnehmer 13, 14 werden in bekannter Weise derart in einer elektronischen Auswerteschaltung verarbeitet, daß ein zur Bestimmung des Meßwertes des Relaxationsgrades ausgewertetes Signal nur dann auftritt, wenn beide Meßwertaufnehmer 13,14 zeitlich definierte Ausganssignale von gleichem Vorzeichen abgeben.

Es erscheint ferner möglich, für die gleichzeitige Elektrostimulation anstelle des Daumens und des kleinen Fingers auch andere Finger der gleichen Hand oder der anderen Hand zu verwenden. Die restlichen Finger der Hand sind jedoch für eine solche Überwachung nur bedingt verwertbar, weil sie durch Muskelgruppen bewegt werden, die nicht im Handbereich liegen. Für Daumen und kleinen Finger liegen die Muskelgruppen im Handbereich und können durch direkte Elektrostimulation der beiden oben genannten Nerven mit besonders günstigen Stimulationspunkten im Handgelenkbereich gereizt werden.

## Ansprüche

1.  Vorrichtung zur Messung des Relaxationsgrades durch Erfassung der durch Elektrostimulation induzierten Muskelkontraktionen im Fingerbereich unter Verwendung von mindestens einem Meßwertaufnehmer, welcher an dem stimulierten Fingerglied befestigbar ist und dessen Ausgangssignal ein Maß für den zu bestimmenden Relaxationsgrad darstellt, **dadurch gekennzeichnet,** daß je ein dem Daumen und dem kleinen Finger der Hand zugeordneter Meßwertaufnehmer (13,14) vorgesehen ist, und daß der Relaxationsgrad aus den kombinierten Ausgangssignalen beider Meßwertaufnehmer (13,14) bestimmt wird.

2. Relaxometriesensor nach Anspruch 1, **dadurch gekennzeichnet,** daß beide Meßwertaufnehmer (13,14) an einem gemeinsamen Träger (1) angeordnet sind.

3. Relaxometriesensor nach Anspruch 2, **dadurch gekennzeichnet,** daß der gemeinsame Träger (1) eine Handgelenkmanschette (4) und einen die Meßwertaufnehmer (13,14) tragenden Handteil (2) aufweist.

4. Relaxometriesensor nach Anspruch 3, **dadurch gekennzeichnet,** daß zwischen dem Handteil (2) des Trägers (1) und der Handgelenkmanschette (4) ein flexibles Übergangsstück (3) angeordnet ist.

5. Relaxometriesensor nach Anspruch 4, **dadurch gekennzeichnet,** daß an der Handgelenkmanschette (4) Stimulationselektroden (5,6) angeordnet sind.

6. Relaxometriesensor nach Anspruch 5, **dadurch gekennzeichnet,** daß der Handteil (2) aus miteinander verbundenen Handrücken- und Handinnenflächenteilen besteht, welche Durchtrittsaussparungen zur Aufnahme des Daumens und der Finger aufweisen.

7. Relaxometriesensor nach Anspruch 1, **dadurch gekennzeichnet,** daß die Meßwertaufnehmer paarweise angeordnete Dehnungsmeßstreifen (13,14) sind.

8. Relaxometriesensor nach Anspruch 1, **dadurch gekennzeichnet** , daß der mit dem Daumen und der mit dem kleinen Finger zu verbindende Meßwertaufnehmer (13,14) in seiner Halterung derart elastisch ausgebildet ist, daß eine zur Messung des Relaxationsgrades ausreichende der stimulierten Auslenkung graduell entgegenwirkende mechanische Vorspannung auftritt.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | US-A-4 236 528 (A. STANEC et al.) <br> * Spalte 1, Zeilen 16-55; Spalte 3, Zeilen 24-61; Spalte 5, Zeilen 15-68; Figur 1 * <br> - - - | 1,3,6 | A <br> 61 B 9/00 <br> A 61 B 5/22 |
| A <br> Y | <br> IEEE TRANSACTIONS ON BIOMEDICAL ENGINEERING, Band 35, Nr. 12, Dezember 1988, Seiten 1090-1093, New York, US; J. SORAB et al.: "Tactile sensory monitoring of clinician-applied forces during delivery of newborns" <br> * Abschnitt: "System design and construction"; Abbildugnen 2-4 * <br> - - - | 5,8 <br> 1,3,6 | |
| A | IDEM <br> - - - | 2,7 | |
| A | US-A-4 387 723 (J.L. ATLEE et al.) <br> * Spalte 3, Zeile 40 - Spalte 4, Zeile 15; Spalte 4, Zeile 59 - Spalte 5, Zeile 62; Spalte 6, Zeilen 23-56; Abbildungen 1,3,4 * <br> - - - - - | 1,3-5,7,8 | |

**RECHERCHIERTE SACHGEBIETE (Int. Cl.5)**

A 61 B

Der vorliegende Recherchenbericht wurde für alle Patentansprüche erstellt

| Recherchenort | Abschlußdatum der Recherche | Prüfer |
|---|---|---|
| Den Haag | 11 März 91 | FONTENAY P.H.E.V. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus anderen Gründen angeführtes Dokument
............................................................
& : Mitglied der gleichen Patentfamilie, übereinstimmendes Dokument